# EUROPEAN PATENT SPECIFICATION

(11) **EP 1 411 064 B1**
(45) Date of publication and mention of the grant of the patent: **14.11.2007**
(21) Application number: 02759752.5
(22) Date of filing: 08.04.2002
(51) Int. Cl.: C07K 16/30

(54) **GANGLIOSIDE-ASSOCIATED RECOMBINANT ANTIBODIES AND THE USE THEREOF IN THE DIAGNOSIS AND TREATMENT OF TUMOURS**
GANGLIOSID-ASSOZIIERTE REKOMBINANTE ANTIKÖRPER UND DEREN VERWENDUNG BEI DER DIAGNOSE UND BEHANDLUNG VON TUMOREN
ANTICORPS DE RECOMBINAISON ASSOCIES A DES GANGLIOSIDES ET LEUR UTILISATION DANS LE DIAGNOSTIC ET LE TRAITEMENT DES TUMEURS

(30) Priority: 06.04.2001 CU 842001
(43) Date of publication of application: 21.04.2004
(60) Divisional application: 06076643.3
(73) Proprietor: Centro de Inmunologia Molecular, 12100 Ciudad Habana (CU)
(72) Inventor: MATEO DE ACOSTA DEL RIO, Cristina Maria, Ciudad de la Habana 10800 (CU); LOMBARDERO VALLADARES, Josefa Agustina 70 entre San, 10 de Octubre Ciudad de la Habana 1050 (CU); ROQUE NAVARRO, Lourdes Tatiana, Ciudad de la Habana 11300 (CU); LOPEZ REQUENA, Alejandro, Av. de Acosta 210(bajos), 10 de Octubre, Ciud. de la Habana 10500 (CU)
(74) Representative: Prins, Adrianus Willem
(86) International application number: PCT/CU2002/000003
(87) International publication number: WO 2002/081496

(56) References cited:
- EP-A- 0 657 471
- EP-A- 0 699 755
- US-A- 5 589 573
- US-B1- 6 207 815
- PEREZ A ET AL: "Immunogenetic analysis of variable regions encoding AB1 and gamma-Type AB2 antibodies from the NeuGc-containing ganglioside family." HYBRIDOMA, vol. 20, no. 4, 2001, pages 211-221, XP002902755
- HUANG D.-B. ET AL: "Comparison of crystal structures of two homologous proteins: Structural origin of altered domain interactions in immunoglobulin light-chain dimers." BIOCHEMISTRY, vol. 33, no. 49, 1994, pages 14848-14857, XP002902756
- KAVALER J ET AL: "A set of closely related antibodies dominates the primary antibody response to the antigenic site CB of the A/PR/8/34 influenza virus hemagglutinin." THE JOURNAL OF IMMUNOLOGY, vol. 145, no. 7, 1 October 1990 (1990-10-01), pages 2312-2321, XP002902757 ISSN: 0022-1767
- GEORGE P. SMITH: "Sequence of the full-lenght immunoglobulin kappa-chain of mouse myeloma MPC 11." BIOCHEMISTRY JOURNAL, vol. 171, no. 2, 1978, pages 337-347, XP002902758

### SEQUENCE LISTING

<110> Centro de Inmunologia Molecular
<120> Ganglioside-associated recombinant antibodies and the use thereof in the diagnosis and treatment of tumors
<130> P66417EP00
<140> EP02759752.5
   <141> 2002-04-08
<150> CU 84/2001
   <151> 2001-04-06
<160> 59
<170> PatentIn version 3.3
<210> 1
   <211> 5
   <212> PRT
   <213> Mus musculus
<400> 1
   Arg Tyr Ser Val His
   1               5
<210> 2
   <211> 16
   <212> PRT
   <213> Mus musculus
<400> 2
   Met Ile Trp Gly Gly Gly Ser Thr Asp Tyr Asn Ser Ala Leu Lys Ser
   1               5                   10                  15
<210> 3
   <211> 14
   <212> PRT
   <213> Mus musculus
<400> 3
   Ser Gly Val Arg Glu Gly Arg Ala Gln Ala Trp Phe Ala Tyr
   1               5                   10
<210> 4
   <211> 11
   <212> PRT
   <213> Mus musculus
<400> 4
   Lys Ala Ser Gln Asp Val Ser Thr Ala Val Ala
   1 5 10
<210> 5
   <211> 7
   <212> PRT
   <213> Mus musculus
<400> 5
   Ser Ala Ser Tyr Arg Tyr Thr
   1 5
<210> 6
   <211> 9
   <212> PRT
   <213> Mus musculus
<400> 6
   Gln Gln His Tyr Ser Thr Pro Trp Thr
   1 5
<210> 7
   <211> 30
   <212> PRT
   <213> Mus musculus
<400> 7
   Gln Val Gln Leu Lys Glu Ser Gly Pro Gly Leu Val Ala Pro Ser Gln
   1 5 10 15
   Ser Leu Ser Ile Thr Cys Thr Val Ser Gly Phe Ser Leu Ser
   20 25 30
<210> 8
   <211> 14
   <212> PRT
   <213> Mus musculus
<400> 8
   Trp Val Arg Gln Pro Pro Gly Lys Gly Leu Glu Trp Leu Gly
   1 5 10
<210> 9
   <211> 32
   <212> PRT
   <213> Mus musculus
<400> 9
   Arg Leu Ser Ile Ser Lys Asp Asn Ser Lys Ser Gln Val Phe Leu Lys
   1               5                   10                  15
   Met Asn Ser Leu Gln Thr Asp Asp Thr Ala Met Tyr Tyr Cys Ala Arg
               20                  25                  30
<210> 10
   <211> 7
   <212> PRT
   <213> Mus musculus
<400> 10
   Trp Gly Gln Gly Thr Leu Val
   1               5
<210> 11
   <211> 23
   <212> PRT
   <213> Mus musculus
<400> 11
   Asp Ile Val Met Thr Gln Ser His Lys Phe Met Ser Thr Ser Val Gly
   1               5                   10                  15
   Asp Arg Val Ser Ile Thr Cys
               20
<210> 12
   <211> 15
   <212> PRT
   <213> Mus musculus
<400> 12
   Trp Tyr Gln Gln Lys Pro Gly Gln Ser Pro Lys Leu Leu Ile Tyr
   1               5                   10                  15
<210> 13
   <211> 32
   <212> PRT
   <213> Mus musculus
<400> 13
   Gly Val Pro Asp Arg Phe Thr Gly Ser Gly Ser Gly Thr Asp Phe Thr
   1 5 10 15
   Phe Thr Ile Ser Ser Val Gln Ala Glu Asp Leu Ala Val Tyr Tyr Cys
   20 25 30
<210> 14
   <211> 7
   <212> PRT
   <213> Mus musculus
<400> 14
   Phe Gly Gly Gly Thr Lys Leu
   1 5
<210> 15
   <211> 5
   <212> PRT
   <213> Mus musculus
<400> 15
   Ser Tyr Asp Ile Asn
   1 5
<210> 16
   <211> 17
   <212> PRT
   <213> Mus musculus
<400> 16
   Trp Ile Phe Pro Gly Asp Gly Ser Thr Lys Tyr Asn Glu Lys Phe Lys
   1 5 10 15
   Gly
<210> 17
   <211> 12
   <212> PRT
   <213> Mus musculus
<400> 17
   Glu Asp Tyr Tyr Asp Asn Ser Tyr Tyr Phe Asp Tyr
   1 5 10
<210> 18
   <211> 11
   <212> PRT
   <213> Mus musculus
<400> 18
   Arg Ala Ser Gln Asp Ile Ser Asn Tyr Leu Asn
   1 5 10
<210> 19
   <211> 8
   <212> PRT
   <213> Mus musculus
<400> 19
   Tyr Thr Ser Arg Leu His Ser Gly
   1 5
<210> 20
   <211> 9
   <212> PRT
   <213> Mus musculus
<400> 20
   Gln Gln Gly Asn Thr Leu Pro Trp Thr
   1 5
<210> 21
   <211> 30
   <212> PRT
   <213> Mus musculus
<400> 21
   Gln Val Gln Leu Gln Gln Ser Gly Ala Glu Leu Val Lys Pro Gly Ala
   1 5 10 15
   Ser Val Lys Leu Ser Cys Lys Ala Ser Gly Tyr Thr Phe Thr
   20 25 30
<210> 22
   <211> 14
   <212> PRT
   <213> Mus musculus
<400> 22
   Trp Val Arg Gln Arg Pro Glu Gln Gly Leu Glu Trp Ile Gly
   1 5 10
<210> 23
   <211> 32
   <212> PRT
   <213> Mus musculus
<400> 23
   Lys Ala Thr Leu Thr Thr Asp Lys Ser Ser Ser Thr Ala Tyr Met Gln
   1 5 10 15
   Leu Ser Arg Leu Thr Ser Glu Asp Ser Ala Val Tyr Phe Cys Ala Arg
   20 25 30
<210> 24
   <211> 9
   <212> PRT
   <213> Mus musculus
<400> 24
   Trp Gly Gln Gly Thr Thr Leu Thr Val
   1 5
<210> 25
   <211> 23
   <212> PRT
   <213> Mus musculus
<400> 25
   Asp Ile Gln Met Thr Gln Thr Thr Ser Ser Leu Ser Ala Ser Leu Gly
   1 5 10 15
   Asp Arg Val Thr Ile Ser Cys
   20
<210> 26
   <211> 15
   <212> PRT
   <213> Mus musculus
<400> 26
   Trp Tyr Gln Gln Lys Pro Asp Gly Thr Val Lys Leu Leu Ile Tyr
   1 5 10 15
<210> 27
   <211> 31
   <212> PRT
   <213> Mus musculus
<400> 27
   Val Pro Ser Arg Phe Ser Gly Ser Gly Ser Gly Thr Asp Tyr Ser Leu
   1 5 10 15
   Thr Ile Ser Asn Leu Glu Gln Glu Asp Ile Ala Thr Tyr Phe Cys
   20 25 30
<210> 28
   <211> 10
   <212> PRT
   <213> Mus musculus
<400> 28
   Phe Gly Gly Gly Thr Lys Leu Glu Ser Lys
   1 5 10
<210> 29
   <211> 39
   <212> DNA
   <213> Artificial sequence
<220>
   <223> primer MAb P3 for VH
<400> 29
   aggtctagaa yctccacaca caggrrccag tggatagac 39
<210> 30
   <211> 29
   <212> DNA
   <213> Artificial Sequence
<220>
   <223> primer MAb P3 VK
<400> 30
   gcgtctagaa ctggatggtg ggaagatgg 29
<210> 31
   <211> 39
   <212> DNA
   <213> Artificial Sequence
<220>
   <223> Primer 1 (signal peptide) for VH
<400> 31
   ggggatatcc accatggrat gsagctgkgt matsctctt 39
<210> 32
   <211> 30
   <212> DNA
   <213> Artificial sequence
<220>
   <223> Primer 2 (CH1) for VH
<400> 32
   ggggctagct gcagagacag tgaccagagt 30
<210> 33
   <211> 39
   <212> DNA
   <213> Artificial Sequence
<220>
   <223> Primer 1 (signal peptide) for VK
<400> 33
   ggggatatcc accatggagw cacakwctca ggtctttrt 39
<210> 34
   <211> 35
   <212> DNA
   <213> Artificial sequence
<220>
   <223> Primer 2 (Ck) for VK
<400> 34
   agcgtcgact tacgtttkat ttccarcttk gtccc 35
<210> 35
   <211> 42
   <212> DNA
   <213> Artificial sequence
<220>
   <223> Primer 1 Humanized Antibody P3
<400> 35
   atgacccagt ctccttcttc tctttccgcg tcagtaggag ac 42
<210> 36
   <211> 42
   <212> DNA
   <213> Artificial sequence
<220>
   <223> Primer 3 Humanized Antibody P3
<400> 36
   gtctcctact gacgcggaaa gagaagaagg agactgggtc at 42
<210> 37
   <211> 30
   <212> DNA
   <213> Artificial sequence
<220>
   <223> Primer MAb 1E10 for VH
<400> 37
   ggggctagct gaggagactg tgagagtggt 30
<210> 38
   <211> 40
   <212> DNA
   <213> Artificial Sequence
<220>
   <223> Primer 1 (signal peptide) for VK
<400> 38
   ggggttaacc accatgaggk ccccwgctca gytyctkggr 40
<210> 39
   <211> 27
   <212> DNA
   <213> Artificial sequence
<220>
   <223> Primer 1 Humanized Antibody 1E10 position 5
<400> 39
   caggttcagc tggtgcagtc tggagct 27
<210> 40
   <211> 27
   <212> DNA
   <213> Artificial sequence
<220>
   <223> Primer 3 Humanized Antibody 1E10 position 5
<400> 40
   agctccagac tgcaccagct gaacctg 27
<210> 41
   <211> 33
   <212> DNA
   <213> Artificial Sequence
<220>
   <223> Primer 1 Humanized Antibody 1E10 position 40 & 42
<400> 41
   tgggtgaggc aggcgcctgg gcagggactt gag 33
<210> 42
   <211> 33
   <212> DNA
   <213> Artificial sequence
<220>
   <223> Primer 3 Humanized Antibody 1E10 position 40 & 42
<400> 42
   ctcaagtccc tgcccaggcg cctgcctcac cca 33
<210> 43
   <211> 27
   <212> DNA
   <213> Artificial Sequence
<220>
   <223> Primer 1 Humanized Antibody 1E10 position 87
<400> 43
   ctcagcaggc tgcggtctga ggactct 27
<210> 44
   <211> 27
   <212> DNA
   <213> Artificial Sequence
<220>
   <223> Primer 3 Humanized Antibody 1E10 position 87
<400> 44
   agagtcctca gaccgcagcc tgctgag 27
<210> 45
   <211> 51
   <212> DNA
   <213> Artificial sequence
<220>
   <223> Primer 1 Humanized Antibody VK1E10 position 7, 8 & 15
<400> 45
   cagatgacac agtctccttc ctccctgtct gcctctgtgg gagacagagt c 51
<210> 46
   <211> 35
   <212> DNA
   <213> Artificial Sequence
<220>
   <223> Primer 2 Humanized Antibody VK1E10 position 7, 8 & 15
<400> 46
   agcgtcgact tacgtttkat ttccarcttk gtccc 35
<210> 47
   <211> 51
   <212> DNA
   <213> Artificial sequence
<220>
   <223> Primer 3 Humanized Antibody VK1E10 position 7, 8 & 15
<400> 47
   gactctgtct cccacagagg cagacaggga ggaaggagac tgtgtcatct g 51
<210> 48
   <211> 354
   <212> DNA
   <213> Mus musculus
<220>
   <221> CDS
   <222> (1)..(354)
   <223> VHP3
<400> 48
   cag gtg cag ctg aag gag tca gga cct ggc ctg gtg gca ccc tca cag       48
   Gln Val Gln Leu Lys Glu Ser Gly Pro Gly Leu Val Ala Pro Ser Gln
   1               5                   10                  15
   agc ctg tcc atc aca tgc act gtc tct ggg ttc tca tta tcc aga tat       96
   Ser Leu Ser Ile Thr Cys Thr Val Ser Gly Phe Ser Leu Ser Arg Tyr
               20                  25                  30
   agt gta cac tgg gtt cgc cag cct cca gga aag ggt ctg gag tgg ctg      144
   Ser Val His Trp Val Arg Gln Pro Pro Gly Lys Gly Leu Glu Trp Leu
           35                  40                  45
   gga atg ata tgg ggt ggt gga agc aca gac tat aat tca gct ctc aaa      192
   Gly Met Ile Trp Gly Gly Gly Ser Thr Asp Tyr Asn Ser Ala Leu Lys
       50                  55                  60
   tcc aga ctg agc atc agc aag gac aac tcc aag agc caa gtt ttc tta      240
   Ser Arg Leu Ser Ile Ser Lys Asp Asn Ser Lys Ser Gln Val Phe Leu
   65                  70                  75                  80
   aaa atg aac agt ctg caa act gat gac aca gcc atg tac tac tgt gcc      288
   Lys Met Asn Ser Leu Gln Thr Asp Asp Thr Ala Met Tyr Tyr Cys Ala
                   85                  90                  95
   aga agt ggg gta cga gag gga agg gcc cag gcc tgg ttt gct tac tgg      336
   Arg Ser Gly Val Arg Glu Gly Arg Ala Gln Ala Trp Phe Ala Tyr Trp
               100                 105                 110
   ggc caa ggg act ctg gtc                                              354
   Gly Gln Gly Thr Leu Val
           115
<210> 49
   <211> 118
   <212> PRT
   <213> Mus musculus
<400> 49
   Gln Val Gln Leu Lys Glu Ser Gly Pro Gly Leu Val Ala Pro Ser Gln
   1 5 10 15
   Ser Leu Ser Ile Thr Cys Thr Val Ser Gly Phe Ser Leu Ser Arg Tyr
   20 25 30
   Ser Val His Trp Val Arg Gln Pro Pro Gly Lys Gly Leu Glu Trp Leu
   35 40 45
   Gly Met Ile Trp Gly Gly Gly Ser Thr Asp Tyr Asn Ser Ala Leu Lys
   50 55 60
   Ser Arg Leu Ser Ile Ser Lys Asp Asn Ser Lys Ser Gln Val Phe Leu
   65 70 75 80
   Lys Met Asn Ser Leu Gln Thr Asp Asp Thr Ala Met Tyr Tyr Cys Ala
   85 90 95
   Arg Ser Gly Val Arg Glu Gly Arg Ala Gln Ala Trp Phe Ala Tyr Trp
   100 105 110
   Gly Gln Gly Thr Leu Val
   115
<210> 50
   <211> 312
   <212> DNA
   <213> Mus musculus
<220>
   <221> CDS
   <222> (1)..(312)
   <223> VKP3
<400> 50
   gac att gtg atg acc cag tct cac aaa ttc atg tcc aca tca gta gga      48
   Asp Ile Val Met Thr Gln Ser His Lys Phe Met Ser Thr Ser Val Gly
   1               5                   10                  15
   gac agg gtc agc atc acc tgc aag gcc agt cag gat gtg agt act gct      96
   Asp Arg Val Ser Ile Thr Cys Lys Ala Ser Gln Asp Val Ser Thr Ala
               20                  25                  30
   gta gcc tgg tat caa cag aaa cca gga caa tct cct aaa cta ctg att      144
   Val Ala Trp Tyr Gln Gln Lys Pro Gly Gln Ser Pro Lys Leu Leu Ile
           35                  40                  45
   tac tcg gca tcc tac cgg tac act gga gtc cct gat cgc ttc act ggc      192
   Tyr Ser Ala Ser Tyr Arg Tyr Thr Gly Val Pro Asp Arg Phe Thr Gly
       50                  55                  60
   agt gga tct ggg acg gat ttc act ttc acc atc agc agt gtg cag gct      240
   Ser Gly Ser Gly Thr Asp Phe Thr Phe Thr Ile Ser Ser Val Gln Ala
   65                  70                  75                  80
   gaa gac ctg gca gtt tat tac tgt cag caa cat tat agt act ccg tgg      288
   Glu Asp Leu Ala Val Tyr Tyr Cys Gln Gln His Tyr Ser Thr Pro Trp
                   85                  90                  95
   acg ttc ggt gga ggc acc aag ctg                                      312
   Thr Phe Gly Gly Gly Thr Lys Leu
               100
<210> 51
   <211> 104
   <212> PRT
   <213> Mus musculus
<400> 51
   Asp Ile Val Met Thr Gln Ser His Lys Phe Met Ser Thr Ser Val Gly
   1               5                   10                  15
   Asp Arg Val Ser Ile Thr Cys Lys Ala Ser Gln Asp Val Ser Thr Ala
               20                  25                  30
   Val Ala Trp Tyr Gln Gln Lys Pro Gly Gln Ser Pro Lys Leu Leu Ile
           35                  40                  45
   Tyr Ser Ala Ser Tyr Arg Tyr Thr Gly Val Pro Asp Arg Phe Thr Gly
       50                  55                  60
   Ser Gly Ser Gly Thr Asp Phe Thr Phe Thr Ile Ser Ser Val Gln Ala
   65                  70                  75                  80
   Glu Asp Leu Ala Val Tyr Tyr Cys Gln Gln His Tyr Ser Thr Pro Trp
                   85                  90                  95
   Thr Phe Gly Gly Gly Thr Lys Leu
<210> 52
   <211> 118
   <212> PRT
   <213> Homo sapiens
<400> 52
   Gln Val Glu Leu Val Glu Ser Gly Gly Gly Val Val Glx Pro Gly Arg
   1 5 10 15
   Ser Leu Arg Leu Ser Cys Ala Ala Ser Gly Phe Thr Phe Ser Asn Tyr
   20 25 30
   Ala Met His Trp Val Arg Gln Pro Pro Gly Lys Gly Leu Glu Trp Val
   35 40 45
   Ala Val Ile Ser Tyr Asx Gly Asx Asx Lys Tyr Tyr Ala Asx Ser Val
   50 55 60
   Lys Gly Arg Phe Thr Ile Ser Arg Asp Asx Ser Lys Asx Thr Leu Tyr
   65 70 75 80
   Leu Gln Met Asn Ser Leu Arg Ala Glu Asx Thr Ala Val Tyr Tyr Cys
   85 90 95
   Ala Arg Asp Arg Pro Leu Tyr Gly Asx Tyr Arg Ala Phe Asn Tyr Trp
   100 105 110
   Gly Gln Gly Thr Leu Val
   115
<210> 53
   <211> 103
   <212> PRT
   <213> Homo sapiens
<400> 53
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Thr Asn Tyr
   20 25 30
   Asn Trp Phe Gln Gln Arg Pro Gly Gln Ala Pro Lys Val Leu Ile Tyr
   35 40 45
   Gly Ala Ser Ile Leu Glu Thr Gly Val Thr Ser Arg Phe Ser Gly Ser
   50 55 60
   Gly Ser Gly Thr Asp Phe Thr Phe Thr Ile Ser Ser Leu Gln Pro Glu
   65 70 75 80
   Asp Ile Ala Thr Tyr Tyr Cys Gln Gln Tyr Asp Thr Leu Pro Leu Thr
   85 90 95
   Phe Gly Gly Gly Thr Lys Val
   100
<210> 54
   <211> 357
   <212> DNA
   <213> Mus musculus
<220>
   <221> CDS
   <222> (1)..(357)
   <223> VH 1E10
<400> 54
   cag gtt cag ctg cag cag tct gga gct gaa ctg gta aag cct ggg gct 48
   Gln Val Gln Leu Gln Gln Ser Gly Ala Glu Leu Val Lys Pro Gly Ala
   1 5 10 15
   tca gtg aag ttg tcc tgc aag gct tct ggc tac acc ttc aca agc tat 96
   Ser Val Lys Leu Ser Cys Lys Ala Ser Gly Tyr Thr Phe Thr Ser Tyr
   20 25 30
   gat ata aac tgg gtg agg cag agg cct gaa cag gga ctt gag tgg att 144
   Asp Ile Asn Trp Val Arg Gln Arg Pro Glu Gln Gly Leu Glu Trp Ile
   35 40 45
   gga tgg att ttt cct gga gat ggt agt act aag tac aat gag aag ttc 192
   Gly Trp Ile Phe Pro Gly Asp Gly Ser Thr Lys Tyr Asn Glu Lys Phe
   50 55 60
   aag ggc aag gcc aca ctg act aca gac aaa tcc tcc agc aca gcc tac 240
   Lys Gly Lys Ala Thr Leu Thr Thr Asp Lys Ser Ser Ser Thr Ala Tyr
   65 70 75 80
   atg cag ctc agc agg ctg aca tct gag gac tct gct gtc tat ttc tgt 288
   Met Gln Leu Ser Arg Leu Thr Ser Glu Asp Ser Ala Val Tyr Phe Cys
   85 90 95
   gca aga gaa gac tac tat gat aac tcc tac tac ttt gac tac tgg ggc 336
   Ala Arg Glu Asp Tyr Tyr Asp Asn Ser Tyr Tyr Phe Asp Tyr Trp Gly
   100 105 110
   caa ggc acc act ctc aca gtc 357
   Gln Gly Thr Thr Leu Thr Val
   115
<210> 55
   <211> 119
   <212> PRT
   <213> Mus musculus
<400> 55
   Gln Val Gln Leu Gln Gln Ser Gly Ala Glu Leu Val Lys Pro Gly Ala
   1               5                   10                  15
   Ser Val Lys Leu Ser Cys Lys Ala Ser Gly Tyr Thr Phe Thr Ser Tyr
               20                  25                  30
   Asp Ile Asn Trp Val Arg Gln Arg Pro Glu Gln Gly Leu Glu Trp Ile
           35                  40                  45
   Gly Trp Ile Phe Pro Gly Asp Gly Ser Thr Lys Tyr Asn Glu Lys Phe
       50                  55                  60
   Lys Gly Lys Ala Thr Leu Thr Thr Asp Lys Ser Ser Ser Thr Ala Tyr
   65                  70                  75                  80
   Met Gln Leu Ser Arg Leu Thr Ser Glu Asp Ser Ala Val Tyr Phe Cys
                   85                  90                  95
   Ala Arg Glu Asp Tyr Tyr Asp Asn Ser Tyr Tyr Phe Asp Tyr Trp Gly
               100                 105                 110
   Gln Gly Thr Thr Leu Thr Val
           115
<210> 56
   <211> 321
   <212> DNA
   <213> Mus musculus
<220>
   <221> CDS
   <222> (1)..(321)
   <223> VK 1E10
<400> 56
   gat atc cag atg aca cag act aca tcc tcc ctg tct gcc tct ctg gga      48
   Asp Ile Gln Met Thr Gln Thr Thr Ser Ser Leu Ser Ala Ser Leu Gly
   1               5                   10                  15
   gac aga gtc acc atc agt tgc agg gca agt cag gac att agc aat tat      96
   Asp Arg Val Thr Ile Ser Cys Arg Ala Ser Gln Asp Ile Ser Asn Tyr
               20                  25                  30
   tta aac tgg tat cag cag aaa cca gat gga act gtt aaa ctc ctg atc      144
   Leu Asn Trp Tyr Gln Gln Lys Pro Asp Gly Thr Val Lys Leu Leu Ile
           35                  40                  45
   tac tac aca tca aga tta cac tca gga gtc cca tca agg ttc agt ggc      192
   Tyr Tyr Thr Ser Arg Leu His Ser Gly Val Pro Ser Arg Phe Ser Gly
       50                  55                  60
   agt ggg tct gga aca gat tat tct ctc acc att agc aac ctg gag caa      240
   Ser Gly Ser Gly Thr Asp Tyr Ser Leu Thr Ile Ser Asn Leu Glu Gln
   65                  70                  75                  80
   gaa gat att gcc act tac ttt tgc caa cag ggt aat acg ctt ccg tgg      288
   Glu Asp Ile Ala Thr Tyr Phe Cys Gln Gln Gly Asn Thr Leu Pro Trp
                   85                  90                  95
   acg ttc ggt gga ggc acc aag ctg gaa atc aaa                          321
   Thr Phe Gly Gly Gly Thr Lys Leu Glu Ile Lys
               100                 105
<210> 57
   <211> 107
   <212> PRT
   <213> Mus musculus
<400> 57
   Asp Ile Gln Met Thr Gln Thr Thr Ser Ser Leu Ser Ala Ser Leu Gly
   1               5                   10                  15
   Asp Arg Val Thr Ile Ser Cys Arg Ala Ser Gln Asp Ile Ser Asn Tyr
               20                  25                  30
   Leu Asn Trp Tyr Gln Gln Lys Pro Asp Gly Thr Val Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asn Thr Leu Pro Trp
                   85                  90                  95
   Thr Phe Gly Gly Gly Thr Lys Leu Glu Ile Lys
               100                 105
<210> 58
   <211> 126
   <212> PRT
   <213> Homo sapiens
<400> 58
   Gln Thr Gln Leu Val Gln Ser Gly Ala Glu Val Arg Lys Pro Gly Ala
   1               5                   10                  15
   Ser Val Arg Val Ser Cys Lys Ala Ser Gly Ile Thr Phe Ile Asp Ser
               20                  25                  30
   Tyr Ile His Trp Ile Arg Gln Ala Pro Gly His Gly Leu Glu Trp Val
           35                  40                  45
   Gly Trp Ile Asn Pro Asn Ser Gly Gly Pro Asn Tyr Ala Pro Arg Phe
       50                  55                  60
   Gln Gly Arg Val Thr Met Thr Arg Asp Ala Ser Phe Ser Thr Ala Tyr
   65                  70                  75                  80
   Met Asp Leu Arg Ser Leu Arg Ser Asp Asp Ser Ala Val Phe Tyr Cys
                   85                  90                  95
   Ala Lys Ser Asp Pro Phe Trp Ser Asp Tyr Tyr Asn Phe Asp Tyr Ser
               100                 105                 110
   Tyr Thr Leu Asp Val Trp Gly Gln Gly Thr Thr Val Thr Val
           115                 120                 125
<210> 59
   <211> 107
   <212> PRT
   <213> Homo sapiens
<400> 59
   Asp Ile Gln Met Thr Glx Ser Pro Ser Ser Leu Ser Ala Ser Val Gly
   1               5                   10                  15
   Asx Arg Val Thr Ile Thr Cys Arg Ala Ser Glx Thr Ile Ser Ser Tyr
               20                  25                  30
   Leu Asx Trp Tyr Glx Glx Lys Pro Gly Lys Ala Pro Asx Leu Leu Ile
           35                  40                  45
   Tyr Ala Ala Ser Asx Leu His Ser Gly Val Pro Ser Arg Phe Ser Gly
       50                  55                  60
   Ser Gly Ser Gly Thr Asx Phe Thr Phe Thr Ile Ser Ser Leu Glx Pro
   65                  70                  75                  80
   Glx Asx Phe Ala Thr Tyr Tyr Cys Glx Glx Ser Tyr Ser Ser Pro Thr
                   85                  90                  95
   Thr Phe Gly Glx Gly Thr Arg Leu Glx Ile Lys
               100                 105

## Description

### Technical Field

The present invention is related to the biotechnology field, in particular with new recombinant antibodies obtained by genetic engineering, specifically with chimeric and humanized antibodies obtained from the murine monoclonal antibody P3 (MAb P3).

More specifically, the invention is related with antibodies that bind to gangliosides containing N-glycolylated sialic acid, but not with the acetylated forms of the gangliosides neither with neuter glycolipids. Gangliosides containing N-glycolylated sialic acid are antigens widely expressed in breast cancer and melanomas. On the other hand, the anti-tumor effect of the MAbai 1E10 has also been demonstrated in experimental models.

The present invention is also related with the pharmaceutical compositions that contain the previously described recombinant antibodies useful in the diagnosis and therapy of cancer, particularly breast cancer and melanomas.

### Prior Art.

Gangliosides are glycosphingolipids that contain sialic acid and they are present in the plasmatic membrane of cells of vertebrates (Stults et al. (1989): Glycosphingolipids: structure, biological source and properties, Methods Enzymology, 179:167-214). Some of these molecules have been reported in the literature as antigens associated to tumors or tumor markers (Hakomori et al. (1991): Possible functions of tumor associated carbohydrate antigens, Curr. Opin. Immunol., 3: 646-653), for that reason the use of anti-gangliosides antibodies has been described as useful in the diagnosis and therapy of cancer (Hougton et al. (1985): Mouse monoclonal antibody IgG3 antibody detecting GD3 ganglioside: to phase I trial in patients with malignant melanoma, PNAS USA, 82:1242-1246; Zhang et al. (1997): Selection of carbohydrate tumor antigens as targets for immune attack using immunohistochemistry. 1. Focus on gangliosides, Int. J. Cancer, 73: 42-49).

Sialic acids more frequently expressed in animals are N-acetyl (NeuAc) and N-glycolyl (NeuGc) (Corfield et al. (1982): Occurrence of sialic acids, Cell. Biol. Monogr., 10: 5-50). NeuGc is not expressed in normal human and chickens tissues in general, but it is broadly distributed in other vertebrates (Leeden and Yu, (1976): Chemistry and analysis of sialic acid. In: Biological Role of Sialic Acid. Rosemberg A and Shengtrund CL (Eds). Plenum Press, New York, 1-48; Kawai et al. (1991): Quantitative determination of N-glycolylneuraminic acid expression in human cancerous tissues and avian lymphoma cell lines as a tumor associated sialic acid by gas chromatography-mass spectrometry, Cancer Research, 51: 1242-1246). However, there are reports that show that antibodies anti-NeuGc, recognize some human tumors and tumor cell lines (Higashi et al. (1988): Detection of gangliosides as N-glycotylneuraminic acid specific tumor-associated Hanganutziu-Deicher antigen in human retinoblastoma cells, Jpn. J. Cancer Res., 79: 952-956; Fukui et al. (1989): Detection of glycoproteins as tumor associated Hanganutziu-Deicher antigen in human gastric cancer cell line, NUGC4, Biochem. Biophys. Res. Commun., 160: 1149-1154). Increased levels of GM3 (NeuGc) gangliosides have been found in human breast cancer (Marquina et al. (1996): Gangliosides expressed in human breast cancer, Cancer Research, 1996; 56: 5165-5171), this result makes attractive the use of this molecule as target for cancer therapy.

The monoclonal antibody (Mab) P3 produced by the cell line deposited with accession number ECACC 94113026 (European Patent EP 0 657 471 B1), it is a murine monoclonal antibody with IgM isotype, that was obtained when fusing murine splenocytes from a BALB/c mouse immunized with liposomes containing GM3(NeuGc) and tetanic toxoid, with the cell line P3-X63-Ag8.653; which is a murine myeloma. This Mab P3 reacts strongly with gangliosides containing N-glycolylated sialic acid but not with the acetylated forms of the gangliosides neither with the neuter glycolipids. It was demonstrated by immunocytochemical and immunohistochemical studies carried out with cell lines and tissues from benign and neoplasic tumors that the Mab P3 recognizes breast cancer (Vázquez et al. (1995): Generation of a murine monoclonal antibody specific for N-glycolylneuraminic acid-containing gangliosides that also recognizes sulfated glycolipids, Hybridoma, 14: 551-556) and melanoma.

The Mab P3 induced anti-idiotypic immune response (Ab2) in mice BALB/c (syngeneic model), even without adjuvant and carrier protein, (Vázquez et al. (1998): Syngeneic anti-idiotypic monoclonal antibodies to an anti-NeuGc-containing ganglioside monoclonal antibody, Hybridoma, 17: 527-534). The role of the electronegative groups, sialic acid (for gangliosides) and SO₃- (for sulfatides), in the recognition properties of this antibody was suggested by immunochemical analysis (Moreno et al. (1998): Delineation of epitope recognized by an antibody specific for N-glycolylneuraminic acid-containing gangliosides, Glycobiology, 8: 695-705).

The anti-idiotypic Mab 1E10 (Mabai 1E10) of subtype IgG1, was obtained from a mouse BALB/c immunized with the Mab P3 coupled to KLH (US Patent 6,063,379, cell line deposited under accession number ECACC 97112901). Mabai 1E10 recognized specifically the MAb P3 and it did not bind other IgM anti-ganglioside antibodies. Moreover, Mabai 1E10 inhibited the specific binding of Mab P3 to the GM3(NeuGc) and to a cell line MDA-MB-435 derived from ductal breast carcinoma (positive for Mab P3 binding). The MAbai 1E10 induced a strong immune response of Ab3 antibodies when mice from syngeneic or alogenic models were immunized, these Ab3 antibodies didn't exhibit the same specificity as the Mab P3 eve when they carry idiotopes similar to those carried by the Ab1 antibody (Vázquez et al. (1998): Syngeneic anti-idiotypic monoclonal antibodies to an anti-NeuGc-containing ganglioside monoclonal antibody, Hybridoma, 17: 527-534). MAbai 1E10 induced a strong antitumor effect in syngeneic as well as alogenic mice. The growth of the mammary carcinoma cell line F3II was significantly reduced by the repeated dose of the MAbai 1E10 coupled KLH in Freund's adjuvant, when BALB/c mice were vaccinated. Also the number of spontaneous lung metastasis was reduced after the vaccination. The intravenous administration of the Mabai 1E10 to C57BL/6 mice inoculated, 10 to 14 days after the intravenous inoculation of melanoma cells B16, caused a dramatic reduction of the number of lung metastases when compared with mice treated with an irrelevant IgG. These results suggest that more than one mechanism of antitumor effect is triggered (Vázquez et al. (2000): Antitumor properties of an anti-idiotypic monoclonal antibody in relation to N-glycolyl-containing gangliosides, Oncol. Rep., 7: 751-756, 2000).

Even when hybridoma technology has been developed during 15 years (Koehler and Milstein (1975): Continuous cultures of fused cells secreting antibody of predefined specificity, Nature, 256: 495-497) and when monoclonal antibodies are still very useful in diagnosis as well as research they have not demonstrated their therapeutic effectiveness in human. It has been mainly due to their short half-life in blood and to that murine effector functions fail for the human immune system, and also for the human anti-mouse antibody immune response (HAMA response).

Otherwise, genetic engineering technology has revolutionized the potential of the MAb utility, since manipulating immunoglobulin genes it is possible to obtain modified antibodies with reduced antigenicity, as well as to improve its effector functions for the treatment or diagnosis of certain pathologies. Methods for reducing immunoglobulin immunogenicity have as essential objective to diminish the differences between a murine antibody and a human immunoglobulin, without altering the antigen recognition specificity (Morrison and Oi (1989): Genetically engineered antibody molecules, Adv Immunol., 44: 65-92).

Recently several methods have been developed to humanize murine or rat antibodies and, of this way, to reduce the xenogenic immune response against foreign proteins when they are injected in humans. One of the first approach to reduce the antigenicity were the chimeric antibodies, in which the variable domains of the murine protein are inserted in constant domains of human molecules, that exhibit the same specificity but reduced immunogenicity compared to their murine counterparts, human effector functions are preserved by chimeric antibodies, (Morrison et al. (1984): Chimeric human antibody molecules: Mouse antigen-binding domains with human constant region domains, PNAS USA, 81: 6851-6855). Even when chimeric antibodies have the same specificity as the murine counterpart, an immune response to the rodent variable regions is frequently observed.

In an attempt to further reduce the immunogenicity of chimeric antibodies, only the CDRs from the rodent monoclonal antibody have been grafted onto human framework regions and this hybrid variable region expressed with human constant regions (Jones et al. (1986): Replacing the complementary-determining regions in a human antibody with those from a mouse, Nature 321: 522-524; Verhoeyen et al. (1988): Reshaping human antibodies: grafting an antilysozyme activity, Science 239, 1534-1536). However, this approach has several shortcomings: frequently the resulting antibody has decreased affinity and a number of framework residues must be backmutated to the corresponding murine ones to restore binding (Rietchmann et al. (1988): Reshaping human antibodies for therapy, Nature, 332: 323-327; Queen et al. (1989): A humanized antibody that binds to the interleukin 2 receptor, PNAS USA, 86: 10029-10033; Tempest et al. (1991): Reshaping a human monoclonal antibody to inhibit human respiratory syncytial virus infection in vivo, Biotechnology, 9: 266-272). In addition, persisting immunogenicity is frequently observed in the CDR-grafted antibodies.

Mateo and collaborators (US Patent Number US 5 712 120) have described a procedure for reducing immunogenicity of murine antibodies. According to the method, the modifications are restricted to the variable domains and specifically to the murine FRs of chimeric antibodies. Moreover, the replacements are only carried out in those regions of the FRs that have amphipatic sequences and therefore they are potential epitopos recognized by T cells. The method comprises judiciously replacement of few amino acid residues, located in the potential immunogenic epitopes by the corresponding residues from the most homologous human sequence, the amino acids that are mainly responsible for canonical structures and also the residues in the immediate neighbourhood of the CDRs or into the Vernier zone, must be retained.

The resulting antibody retains its antigen binding specificity and to be less immunogenic than either its murine or chimeric predecessor (Mateo et al. (2000): Removal of T cell epitopes from genetically engineered antibodies: Production of modified immunoglobulins with reduced immunogenicity, Hybridoma 19: 463-71), these properties increases their therapeutic utility. Using this new procedure only few mutations, and of course less genetic manipulations, have to be done.

### Detailed description of the Invention

The present invention relates to a chimeric monoclonal antibody derived from the murine monoclonal antibody Mab P3 that recognizes gangliosides containing N-glycolylated sialic acid, and which is produced by the hybridoma cell line with deposit number ECACC 94113026, wherein the hypervariable domains of the heavy and light chains of Mab P3 comprise the following sequences:
**HEAVY CHAIN**
   CDR1: RYSVH
   CDR2: MIWGGGSTDYNSALKS
   CDR3: SGVREGRAQAWFAY
**LIGHT CHAIN**
   CDR1: KASQDVSTAVA
   CDR2: SASYRYT
   CDR3: QQHYSTPWT;
the framework regions (FRs) of the heavy and light chains of Mab P3 comprise the following sequences:
**HEAVY CHAIN**
   FR1: QVQLKESGPGLVAPSQSLSITCTVSGFSLS
   FR2: WVRQPPGKGLEWLG
   FR3: RLSISKDNSKSQVFLKMNSLQTDDTAMYYCAR
   FR4: WGQGTLV
**LIGHT CHAIN**
   FR1: DIVMTQSHKFMSTSVGDRVSITC
   FR2: WYQQKPGQSPKLLIY
   FR3: GVPDRFTGSGSGTDFTFTISSVQAEDLAVYYC
   FR4: FGGGTKL;
wherein the chimeric monoclonal antibody, for its humanization and preservation of the binding properties to the antigen, includes the following substitutions in the FR1 region of the light chain:
**LIGHT CHAIN:**
   Position 8: His by Pro
   Position 9: Lys by Ser
   Position 10: Phe by Ser
   Position 11: Met by Leu
   Position 13: Thr by Ala

In a preferred embodiment of the chimeric monoclonal antibody of the invention, it contains the constant region of heavy chain human IgG1 and the constant region of light chain human Ck. Thus, it is preferred that the constant region of the heavy chain comprises the amino acid sequence of gamma-1 chain and the constant region of the light chain comprises the amino acid sequence of a kappa chain, both derived from human immunoglobulins.

The invention further relates to a cell line that produces a monoclonal antibody of the invention; to a pharmaceutical composition for the treatment of malignant tumors of breast and melanomas, their metastases and relapses which comprises a monoclonal antibody of the invention; to a pharmaceutical composition for *in vivo* localization and identification of malignant tumors-of breast and melanomas, their metastases and relapses which comprises a monoclonal antibody of the invention; and to the use of a monoclonal antibody of the invention for the manufacture of a medicament useful for the treatment of malignant tumours of breast and melanomas, their metastases and relapses. These include tumors of breast, lung, digestive system, urogenital system, melanomas, sarcomas and neuroectodermic tumors, and their metastases and relapses.

### cDNA Synthesis and Gene Amplification by PCR (Polymerase chain reaction) of the variable region of MAb P3 and Mabai 1E10.

Cytoplasmic RNA was extracted from about 10⁶ hybridoma cells of P3 (murine IgM MAb, that recognizes to the GM3 N-glycolylated ganglioside) or of 1E10 (antidiotype anti-P3 antibody). The RNA was extracted by using the reagent TRIZOL (GIBCO BRL, Grand Island, NY), according to the manufacturers instructions.

The cDNA synthesis reaction was carried out mixing 5µg of the RNA, 25 pmoles of Vh (complementary to the constant region of murine IgM for VHP3, and with the constant region of murine IgG1 for VH 1E10) or Vk (complementary to constant murine kappa region for both antibodies), 2.5 mM of each dNTPs, 50 mM Tris-Hcl pH 7.5, 75 mM KCI, 10 mM DTT, 8 mM MgCl2 and 15 units of RNAse inhibitor in a 50 µl reaction mixture. It was heated at 70°C, for 10 minutes and slowly cooled up to 37°C. Then, 100 units of MLV reverse transcriptase enzyme were added and the incubation at 42°C continued for one hour.

The variable regions VK and VH cDNAs were amplified by PCR. Shortly, 5 µl cADN of VH or VK were mixed with 25 pmoles of specific primers, 2.5 mM of each dNTP, 5 µl constituents of 10X buffer Taq DNA polymerase and 1 unit of this enzyme. The samples were subjected to 25 thermal cycles at 94°C, 30 sec; 50°C, 30 sec; 72°C, 1 min, and a last incubation for 5 minutes at 72°C.

### Cloning and Sequencing of Amplified cDNA

The PCRs products of VH and VK (of the P3 and of the 1E10 respectively) were cloned into TA vector (TA Cloning kit. Promega, USA). The resulting clones were sequenced by the dideoxy method using T7 DNA Polymerase (T7 sequencing kit, Pharmacia, Sweden).

### Construction of chimeric genes

The VH VK genes were excised from TA vectors by enzymatic digestion and they were cloned into the respective expression vectors (Coloma et al. (1992): Novel vectors for the expression of antibody molecules using variable regions generated by polymerase chain reaction, J. Immunol. Meth., 152: 89-104).

The VH genes were excised from the TA vector by enzymatic digestion with EcoRV and Nhel and cloned in an expression vector (PAH 4604) that has included a variable region human IgG1 and the histidinol resistance gene. The resultant constructions were P3VH-PAH4604 and 1E10VH-PAH4604. The VK genes were excised from TA vector by enzymatic digestion with EcoRV and Sall and cloned in an expression vector (PAG4622). This vector has included mycophenolic acid resistance gene and the human kappa constant region. The resultant constructions were P3VK-PAG4622 and 1E10VK-PAG4622.

### Expression of chimeric antibodies obtained from Mab P3 and Mabid 1E10.

NS-0 cells were electroporated with 10 µg of P3VK-PAG4622 or 1E10VK-PAG4622, clones expressing human kappa light chains were transfected with 10 µg of P3VH-PAH4604 or 1E10VH-PAH4604.

The DNAs were linearized by digestion with Pvul enzyme, precipitated with ethanol and dissolved in 50 µl of PBS. Approximately 10⁷ cells were harvested by centrifugation and resupended in 0.5 ml of PBS together with the digested DNA in an electroporation cuvette.

After 10 minutes on ice, the cells were given a pulse of 200 volts and 960 µF and left in ice for a further 10 minutes. The cells were distributed into 96 wells plate with D'MEM F12 plus 10% fetal calf serum. Two or four days later, it is added selective medium (D'MEM F12 with mycophenolic acid 0,45 µg/mL or histidinol 10mM, respectively). Transfected clones were visible with the naked eyes 14 days later.

The presence of human antibody in the medium of the wells containing transfected clones was measured by ELISA. Microtiter plate wells were coated with goat anti-human kappa light chain (for human kappa chain producing clones) or anti-human IgG (gamma chain specific) (for the complete antibody producing clones) antibodies. After washing with PBST (saline phosphate buffered solution containing 0.05% Tween 20), diluted culture medium of the wells containing transfectants was added to each microtiter well for one hour at 37°C. The wells were washed with PBS-T and peroxidase of spicy radish-conjugated goat anti-human kappa light chain or alkaline phosphatase-conjugated goat anti-human IgG (gamma chain specific), were added and incubated at 37°C one hour. The wells were washed with PBS-T and substrate buffer containing o-phenylendiamine or p-nitrophenylphosphate, respectively, was added. After half hour, absorbance at 492 or 405 nm respectively, was measured.

### Construction of the humanized antibodies P3hu and 1E10 hu by humanization of T cell epitopes. Prediction of T cell epitopes

The sequences of P3 and 1E10 variable domains were analysed with the algorithm AMPHI (Margalit et al. (1987): Prediction of immunodominant helper T cell antigenic sites from the primary sequence, J. Immunol., 138: 2213-2229). It searched helical amphipatic segments, with 7 or 11 aminoacid residues, which have been associated with T immunogenicity. The program SOHHA also predicted helical hydrophobic segments. (Elliot et al. (1987). An hypothesis on the binding of an amphipatic, alpha helical sequence in li to the desotope of class 11 antigen, J. Immunol., 138: 2949-2952). Both algorithms predicted which segments from variable region sequences of antibodies P3 and 1E10 could be presented to T-helper cells in the context of MHC class II molecules.

### Homology analysis with human immunoglobulins.

The amino acid sequences of murine variable regions were compared with the immunoglobulin sequences included in the GeneBank and EMBL database (available in Intemet). The most homologous human variable region sequence was determined for each antibody. Software PC-TWO HIBIO PROSIS 06-00 (Hitachi) was used for sequences homology searching.

### Analysis for the immunogenicity reduction.

The aim of the method is to reduce immunogenicity breaking or humanizing potential immunogenic T epitopes, with a minimum of changes. The method comprises judiciously replacement of few amino acid residues, located into helical amphipatic segments. The amino acids, which are mainly responsible for canonical structures and also the residues in the immediate neighbourhood of the CDRs or into Vernier zone, must be retained.

According to the method, murine variable region sequences were compared with the most homologous human sequence and different aminoacid residues at each position between the murine MAb and the most homologous human sequence were identified, only residues into FRs were taken into account (Kabat (1991), Sequences of proteins of immunological interest, Fifth Edition, National Institute of Health), the previously defined residues were replaced by those residues present in the most homologous human sequence. Replacements were made by directed mutagenesis techniques.

Residues involved in three-dimensional structure of the binding site were not mutated; it could affect antigen recognition. Additional information about the influence of the replacements in the tertiary structure can be obtained by molecular modelling of the antigen binding site.

The presence of proline residues into the helical amphipatic segment and the fact that a certain murine residues don't appear in the same position in the most homologous human sequence but be frequent in other human immunoglobulins, must be kept in mind. For that reason there is not a unique ensemble of murine amino acids to be replaced into the frameworks. It is possible to obtain different versions of the modified antibody with different numbers of replacements. The mutations were carried out by over-lapping of PCRs.

### Cloning and expressing humanized antibodies P3hu and 1E10hu.

The genetic constructions corresponding to the P3hu and 1E10hu, were cloned in expression vectors following the method described for the chimeric antibodies. The resultants constructions were P3VKhu-PAG4622 or 1E10Vkhu-PAG4622 and P3VHhu-PAH4604 and 1E10VHhu-PAH4604. They were transfected into NS-0 cells following the protocol described previously for chimeric antibodies.

### Purification of the recombinant antibodies.

The recombinant antibodies were purified by affinity chromatography using protein A (Pharmacia, Upssala, Sweden).

### Biological activity.

The specific binding to antigen measured by ELISA tested the biological activity of the recombinant antibodies.

For recombinant MAb P3, microtiter plates were coated with GM3(NeuGc) ganglioside in methanol. After drying one hour, unspecific binding was blockade with bovine sera albumin (BSA) 1% in Tris-HCI buffer, incubated for one hour at 37°C. The wells were washed with PBS and incubated for 1 hour at 37°C with purified recombinant Mab P3. The wells were washed with tris-HCI and a goat anti- human antibody conjugated with alkaline phosphatase was added and incubated at 37°C for one hour. Finally, the wells were washed and the substrate buffer containing *p*-nitrophenylphosphate was added. After half hour absorbance at 405 or 492 nm respectively, was measured.

For recombinant Mabai 1E10, the ELISA assay was similar, except that wells were coated with Mab P3 and washing were made with PBS-0.05% Tween 20.

### Examples.

In the following examples all the enzymes used, as well as reagents and materials were obtained from commercial sources unless the opposite is specified.

### Example 1. Obtaining of chimeric MAb P3.

The cDNA synthesis was obtained by a reaction with reverse transcriptase enzyme, starting with RNA from the hybridoma producing Mab P3, as described previously. The sequence of the specific primers used in this reaction is shown:
**For VH:**
   5' AGGTCTAGAA(CT)CTCCACACACAGG(AG)(AG)CCAGTGGATAGAC 3'
**For VK:**
   5' GCGTCTAGAACTGGATGGTGGGAAGATGG 3'

cDNA VHP3 and cDNA VKP3 were amplified by PCR using Taq Polymerase and specific primers. The restriction sites included in the primers were ECORV /NHEI, for VH and ECORV/SALI for VK. The primers sequences used were the following:
**For VH:**
   Primer 1 (signal peptide):
      5'GGGGATATCCACCATGG(AG)ATG(CG)AGCTG(TG)GT(CA)AT(CG)CTCTT 3'
   Primer 2 (CH1):
      5' GGGGCTAGCTGCAGAGACAGTGACCAGAGT 3'
**For VK:**
   Primer 1 (signal peptide):
      5' GGGGATATCCACCATGGAG(TA)CACA(GT)(TA)CTCAGGTCTTT(GA)T 3'
   Primer 2 (Ck):
      5' AGCGTCGACTTACGTTT(TG)AT17CCA(GA)CTT(GT)GTCCC 3'

PCR products were cloned into TA vector (TA cloning kit, Invitrogen). Twelve independent clones were sequenced by the dideoxy method using T7 DNA Pol (Pharmacia). By homology search analysis it was determined the most homologous sequence group for VHP3 and

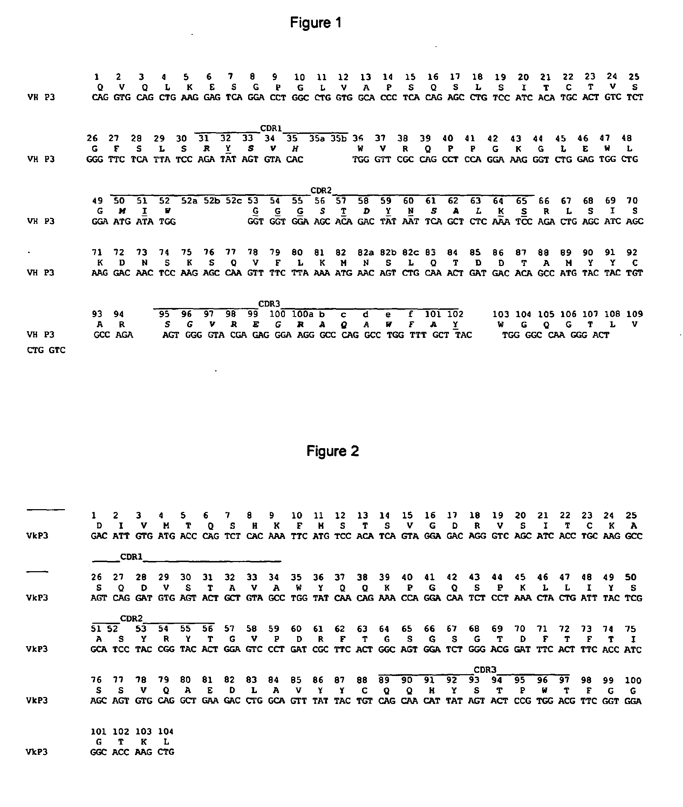

VKP3. VHP3 and VKP3 sequences (Figures 1 and 2) have high homology with groups IB and V respectively according to Kabat's classification.

After digestion with the restriction enzymes ECORV and NHEI for VHP3 and with ECORV and SALI for VKP3, they were cloned in the expression vectors previously digested with the same enzymes, PAH4604 and PAG4622 for VH and VK respectively. These expression vectors were donated by Sherie Morrison (UCLA, California, USA), they are suitable for immunoglobulins expression in mammalian cells. The vector PAH 4604 have included the human constant region IgG1 and the PAG 4622 human (Coloma et al. (1992): Novel vectors for the expression of antibody molecules using variable regions generated by polymerase chain reaction, J. Immunol. Meth., 152: 89-104). The resultant constructs were P3VH-PAH4604 and P3VK-PAG4622.

NS-0 cells were transfected with 10 µg of P3VK-PAG4622, a clone expressing light chain was transfected with 10 µg P3VH-PAH4604, in both cases DNA is linearized with Pvul, ethanol precipitated and dissolved in 50 µl of PBS before transfection.

Approximately 10⁷ cells were harvested by centrifugation and resupended in 0.5 ml of PBS together with the digested DNA in an electroporation cuvette. After 10 minutes on ice, the cells were given a pulse of 200 volts and 960 µF and left in ice for a further 10 minutes. The cells were distributed into 96 wells plate with D'MEM F12 plus 10% fetal calf serum. Two or four days later, it is added selective medium (D'MEM F12 with mycophenolic acid 0,45 ug/mL or histidinol 10mM, respectively). Transfected clones were visible with the naked eyes 14 days later.

The presence of human antibody in the medium of wells containing transfected clones was measured by ELISA. Microtiter plate wells were coated with goat anti-human kappa light chain (for human kappa chain producing clones) or anti-human IgG (gamma chain specific) (for the complete antibody producing clones) antibodies. After washing with PBST (saline phosphate buffered solution containing 0.05% Tween 20), diluted culture medium of the wells containing transfectants was added to each Microtiter well for one hour at 37°C. The wells were washed with PBS-T and peroxidase of spicy radish-conjugated goat anti-human kappa light chain or alkaline phosphatase-conjugated goat anti-human IgG (gamma chain specific), were added and incubated at room temperature one hour. The wells were washed with PBS-T and substrate buffer containing *o*-phenylendiamine or *p*-nitrophenylphosphate, respectively, was added. After half hour absorbance at 492 or 405 nm respectively, was measured.

### Example 2. Obtaining different versions of the Humanized Antibody P3.

Murine VHP3 and VKP3 sequences (Figures 1 and 2) were compared with human sequences. Figures 3 and 4 show the most homologous human sequences. Helical amphipatic regions or potential T cell epitopes were searched on murine P3 variable region sequences and according with the method a judiciously strategy for aminoacid replacements was established in order to break or humanize potential T cell epitopes into the murine sequences.

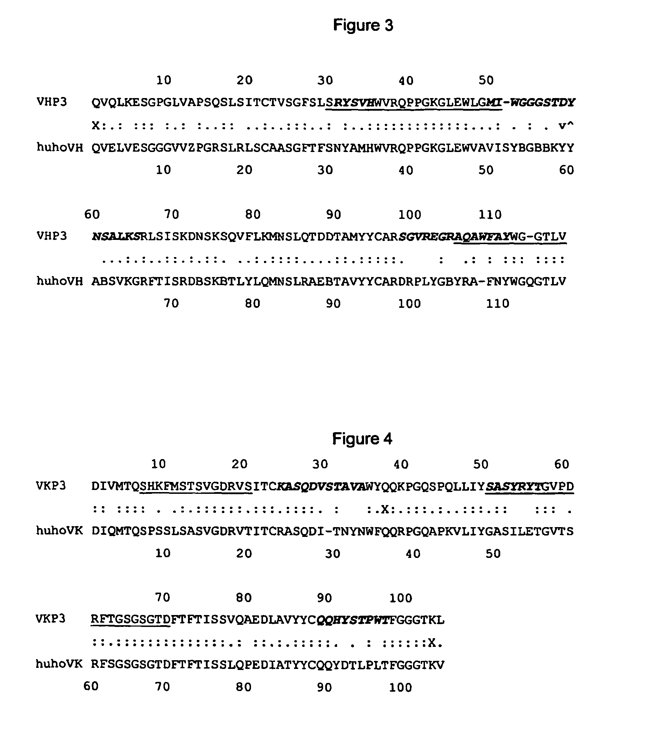

The analysis on VHP3 rendered (Figure 3) 2 amphipatic segments, the first one embraces CDR1, FR2 and some residues of the CDR2, the second one embraces the end of FR3 and CDR3. The main differences of murine sequence in comparison with the most homologous human sequence were founded in CDRs or residues involved with the three dimensional structure of the binding site. For that reason it was decided do not replace any aminoacid in murine VHP3.

The analysis for VKP3 rendered also 2 amphipatic segments (Figure 4), the first segment embraces FR1, the second one embraces CDR2 and some residues of the FR3. It was decide to replace residues at positions 8,9,10,11 and 13 by residues at the same position in the most homologous human sequence. The amino acids His, Lys, Phe, Met and Thr were replaced by Pro, Ser, Ser, Leu, and Ala, respectively. The replacements were made by PCR overlapping (Kammann et al. (1989) Rapid insertional mutagenesis of DNA by polymerase chain reaction (PCR), Nucleic Acids Res., 17: 5404) using primers 1 and 2 and 3 and 4 whose sequences are the following:
Primer 1:
   5' ATGACCCAGTCTCCTTCTTCTCTTTCCGCGTCAGTAGGAGAC 3'
Primer 2:
   5' AGCGTCGACTTACGTTT(TG)ATTTCCA(GA)CTT(GT)GTCCC 3'
Primer 3:
   5' GTCTCCTACTGACGCGGAAAGAGAAGAAGGAGACTGGGTCAT 3'
Primer 4:
   5'GGGGATATCCACCATGGAG(TA)CACA(GT)(TA)CTCAGGTCTTT(GA)T 3'

The point mutations were verified by sequencing. The resultant construct was P3Vkhu and it was cloned in PAG 4622 expression vector. The resultant construct was P3VKhu-PAG4622. To express the humanized antibody P3, NS-0 cells were transfected with P3VH-PAH4604 and P3VKhu-PAG4622

P3hu antibody was transfected following the same procedure of electroporation and detection described previously for the chimeric antibodies.

### Example 3: Biological activity of chimeric MAb P3.

The specific binding to antigen measured by ELISA tested the biological activity of the Mab P3 chimeric.

For recombinant MAb P3, microtiter plates were coated with GM3(NeuGc) ganglioside in methanol. After drying one hour at 37°C, unspecific binding was blockade with bovine sera albumin (BSA) 1% in Tris-HCI buffer, incubated for one hour at 37°C. The wells were washed with PBS and incubated for 1 hour at 37°C with purified recombinant Mab P3. The wells were washed with tris-HCI and a goat anti- human antibody conjugated with alkaline phosphatase was added and incubated at 37°C for one hour. Finally, the wells were washed with Tris-HCI and the substrate buffer containing p-nitrophenylphosphate was added. After half hour absorbance at 405 nm, was measured.

Mab T1 chimeric was used as negative control.

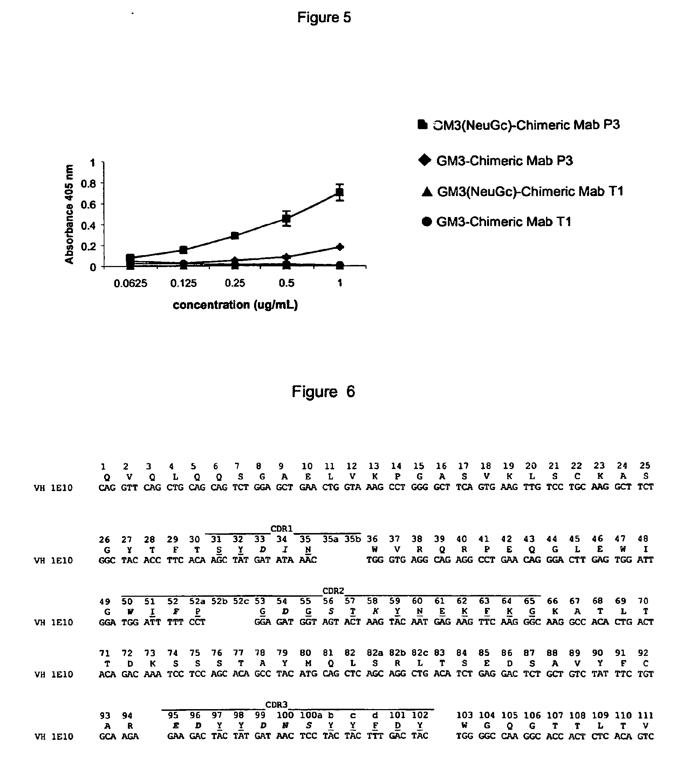

Figure 5 shows the specific binding of Mab P3 chimeric to the antigen.

The following EXAMPLES 4-6 do not concern the invention.

### Example 4. Obtaining of chimeric MAb 1E10.

The cDNA synthesis was obtained by a reaction with reverse transcriptase enzyme, starting with RNA from the hybridoma producing Mab 1E10, as described previously. The sequence of the specific primers used in this reaction is shown following:
**For VH:**
   5'GGCGCTAGCTGAGGAGACTGTGAGAGTGGT 3'
**For VK:**
   5'GCGTCTAGAACTGGATGGTGGGAAGATGGA 3'

cDNA VH1E10 and cDNA VK1E10 were amplified by PCR using Taq Pol and specific primers.
**For VH:**
   Primer 1 (signal peptide):
      5'GGGGATATCCACCATGG(AG)ATG(CG)AGCTG(TG)GT(CA)AT(CG)CTCTT 3'
   Primer 2 (CH1):
      5' GGGGCTAGCTGAGGAGACTGTGAGAGTGGT 3'
**For VK:**
   Primer 1 (signal peptide):
      5'GGGGTTAACCACCATGAGG(GT)CCCC(AT)GCTCAG(CT)T(CT)CT(TG)GG(GA)3'
   Primer 2 (Ck):
      5'AGCGTCGACTTACGTTT(TG)ATTTCCA(GA)CTT(GT)GTCCC3'

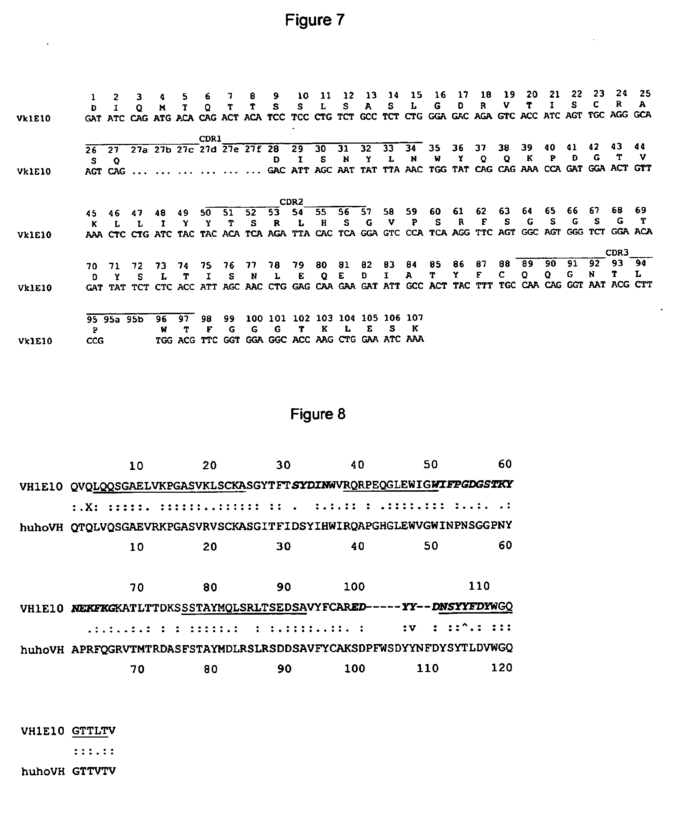

PCR products were cloned into TA vector (TA cloning kit, Invitrogen). Twelve independent clones were sequenced (Figures 7 and 8) by the dideoxy method using T7 DNA Pol (Pharmacia). By homology search analysis it was determined the most homologous sequence group for VH1E10 and VK1E10. VH1E10 and VK1E10 sequences have high homology with groups miscellaneous and V respectively according to Kabat's classification. After digestion with the restriction enzymes ECORV and NHEI for VH1E10 and with HincII and SALI for VK1E10, they were cloned in the expression vectors previously digested with appropriated enzymes, PAH4604 and PAG4622 for VH and VK respectively. These expression vectors were donated by Sherie Morrison (UCLA, California, USA), they are suitable for immunoglobulins expression in mammalian cells. The vector PAH 4604 have included the human constant region IgG1 and the PAG 4622 human (Coloma et al. (1992): Novel vectors for the expression of antibody molecules using variable regions generated by polymerase chain reaction, J. Immunol. Meth., 152: 89-104). The resultant constructs 1E10VH-PAH4604 and 1E10VK-PAG4622.

NS-0 cells were transfected with 10 µg of 1E10VK-PAG4622, a clone expressing light chain was transfected with 10 µg 1E10VH-PAH4604, in both cases DNA is linearized with Pvul, ethanol precipitated and dissolved in 50 ul of PBS before transfection.

Approximately 10⁷ cells were harvested by centrifugation and resupended in 0.5 ml of PBS together with the digested DNA in an electroporation cuvette. After 10 minutes on ice, the cells were given a pulse of 200 volts and 960 µF and left in ice for a further 10 minutes. The cells were distributed into 96 wells plate with D'MEM F12 plus 10% fetal calf serum. Two or four days later, it is added selective medium (D'MEM F12 with mycophenolic acid 0,45 µg/mL or histidinol 10mM, respectively). Transfected clones were visible with the naked eyes 14 days later.

The presence of human antibody in the medium of wells containing transfected clones was measured by ELISA. Microtiter plate wells were coated with goat anti-human kappa light chain (for human kappa chain producing clones) or anti-human IgG (gamma chain specific) (for the complete antibody producing clones) antibodies. After washing with PBST (phosphate buffered saline containing 0.05% Tween 20), diluted culture medium of the wells containing transfectants was added to each Microtiter well for one hour at 37°C. The wells were washed with PBS-T and peroxidase of spicy radish-conjugated goat anti-human kappa light chain or alkaline phosphatase-conjugated goat anti-human IgG (gamma chain specific), were added and incubated at room temperature one hour. The wells were washed with PBS-T and substrate buffer containing *o*-phenylendiamine or *p*-nitrophenylphosphate, respectively, was added. After half hour absorbance at 492 or 405 nm respectively, was measured.

### Example 5. Obtaining different versions of the Humanized Antibody 1E10.

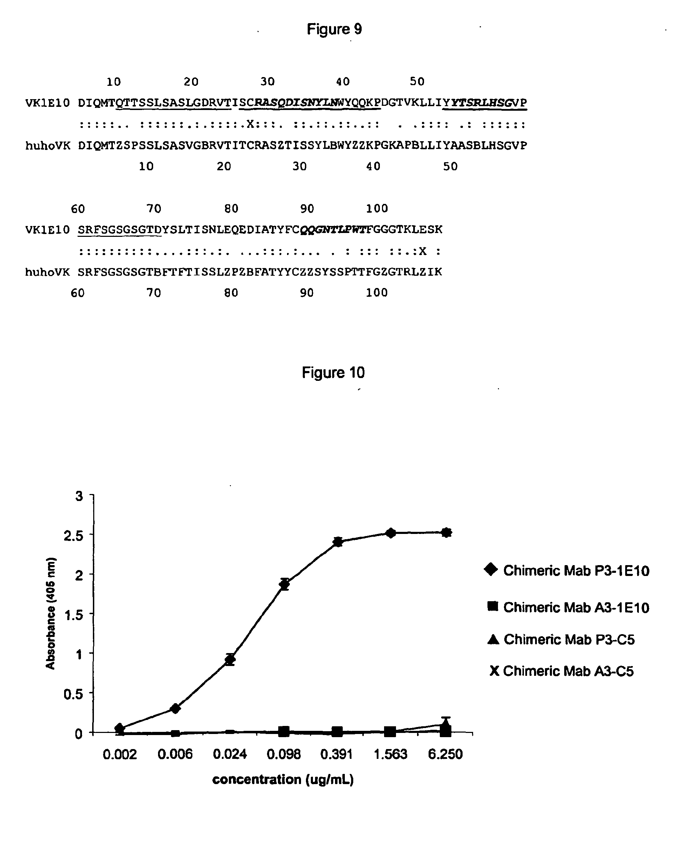

Murine VH1E10 VK1E10 sequences (Figures 6 and 7) were compared with human sequences, Figures 8 and 9 shown the most homologous human sequences. Helical amphipatic regions or potential T cell epitopes were searched on murine 1E10 variable region sequences and according with the method a judiciously strategy for aminoacid replacements was established in order to break or humanize potential T cell epitopes into the murine sequences

The analysis on VH1E10 rendered (Figure 8) 3 amphipatic segments, the first one embraces FR1, the second one embraces FR2, the third one embraces FR3. It was decide to replace residues at positions 5, 40, 42 and 87 (83 according to Kabat's numbering) by residues at the same position in the most homologous human sequence. The amino acids Gln, Arg, Glu and they were replaced by Val, Ala, Gly and Arg, respectively.

The replacements were made by PCR overlapping (Kammann et al. (1989) Rapid insertional mutagenesis of DNA by polymerase chain reaction (PCR), Nucleic Acids Res., 17: 5404) using different set of primers.

Primers for mutation at position 5 of the heavy chain were 1 and 2 and 3 and 4 whose sequences are the following:
Primer 1:
   5' CAGGTTCAGCTGGTGCAGTCTGGAGCT 3'
Primer 2:
   5' GGGGCTAGCTGAGGAGACTGTGAGAGTGGT 3'
Primer 3:
   5' AGCTCCAGACTGCACCAGCTGAACCTG 3'
Primer 4:
   5'GGGGATATCCACCATGG(AG)ATG(CG)AGCTG(TG)GT(CA)AT(CG)CTCTT 3'

After checking by sequence the point mutation at position 5, mutations at positions 40 and 42 were introduced.

Primer for mutations at positions 40 and 42 of the heavy chain:
Primer 1:
   5' TGGGTGAGGCAGGCGCCTGGGCAGGGACTTGAG 3'
Primer 2:
   5' GGGGCTAGCTGAGGAGACTGTGAGAGTGGT 3'
Primer 3:
   5' CTCAAGTCCCTGCCCAGGCGCCTGCCTCACCCA 3'
Primer 4:
   5'GGGGATATCCACCATGG(AG)ATG(CG)AGCTG(TG)GT(CA)AT(CG)CTCTT 3'

After checking by sequence the point mutation at positions 40 and 42, mutation at positions 87 (83 according to Kabat's numbering) was introduced.

Primer for mutations at position 87 (83 according to Kabat's numbering) of the heavy chain:
Primer 1:
   5' CTCAGCAGGCTGCGGTCTGAGGACTCT 3'
Primer 2:
   5' GGGGCTAGCTGAGGAGACTGTGAGAGTGGT 3'
Primer 3:
   5' AGAGTCCTCAGACCGCAGCCTGCTGAG 3'
Primer 4:
   5'GGGGATATCCACCATGG(AG)ATG(CG)AGCTG(TG)GT(CA)AT(CG)CTCTT 3'

Other replacements were not made because residues were involved in the three dimensional structure of the binding site.

The point mutations were verified by sequencing. The resultant construct was 1E10VHhu and it was cloned in PAH4604 expression vector. The resultant construct was 1E10VH-PAH4604.

The analysis for VK1E10 rendered also 3 amphipatic segments (Figure 9), the first segment embraces FR1, the second one embraces CDR1 and the thirst one embraces FR3. It was decide to replace residues at positions 7,8 and 15 by residues at the same position in the most homologous human sequence. The amino acids Thr, Thr and Leu were replaced by Ser, Pro and Val, respectively. The replacements were made by PCR overlapping (Kammann et al. (1989) Rapid insertional mutagenesis of DNA by polymerase chain reaction (PCR), Nucleic Acids Res., 17: 5404) using primers 1 and 2 and 3 and 4 whose sequences are the following:

Primers for mutation at positions 7, 8 and 15 of the light chain:
Primer 1:
   5'CAGATGACACAGTCTCCTTCCTCCCTGTCTGCCTCTGTGGGAGACAGAGTC 3'
Primer 2:
   5'AGCGTCGACTTACGTTT(TG)ATTTCCA(GA)CTT(GT)GTCCC 3'
Primer 3:
   5'GACTCTGTCTCCCACAGAGGCAGACAGGGAGGAAGGAGACTGTGTCATCTG 3'
Primer 4:
   5'GGGGTTAACCACCA TGAGG(GT)CCCC(AT)GCTCAG(CT)T(CT)CT(TG)GG(GA) 3'

The point mutations were verified by sequencing. The resultant construct was 1E10Vkhu and it was cloned in PAG 4622 expression vector. The resultant construct was 1E10VKhu-PAG4622.

To express the humanized antibody 1E10, NS-0 cells were transfected with 1E10VHhu-PAH4604 and 1E10VKhu-PAG4622

1E10hu antibody was transfected following the same procedure of electroporation and detection described previously for the chimeric antibodies.

### Example 6: Biological activity of chimeric MAb1E10.

The specific binding to antigen measured by ELISA tested the biological activity of the Mab 1E10 chimeric.

For recombinant MAb 1E10, Microtiter plates were coated with Mab P3. After washing with PBST (saline phosphate buffered solution containing 0.05% Tween 20), unspecific binding was blockade with bovine sera albumin (BSA) 1% in PBST, incubated for one hour at 37°C. The wells were washed and incubated for 1 hour at 37°C with purified recombinant Mab 1E10. The wells were washed with PBST and a goat anti- human antibody conjugated with alkaline phosphatase was added and incubated at 37⁰C for one hour. Finally, the wells were washed with PBST and the substrate buffer containing p-nitrophenylphosphate was added. After half hour absorbance at 405 nm respectively, was measured.

Mab C5 chimeric was used as negative control.

Figure 10 shows the specific binding of Mab 1E10 chimeric to Mab P3.

### Brief description of the figures:

**Figure 1:** VHP3 DNA and deduced amino acid sequences. Sequences are aligned according Kabat's numbering (Kabat et al. (1991), Sequences of proteins of immunological interest, Fifth Edition, National Institute of Health), CDRs appeared marked with dotted lines.
**Figure 2:** VKP3 DNA and deduced amino acid sequences. Sequences are aligned according Kabat's numbering (Kabat and collaborators (1991), Sequences of proteins of immunological interest, Fifth Edition, National Institute of Health), CDRs appeared marked with dotted lines.
**Figure 3:** VHP3 was aligned with the most homologous human sequence. Amphipatic segments are underlined and CDRs in bold.
**Figure 4:** VKP3 was aligned with the most homologous human sequence. Amphipatic segments are underlined and CDRs in bold.
**Figure 5:** Specific binding to GM3(NeuGc) by chimeric Mab P3. Different concentrations of Mab P3 and MAb T1 (negative control) were tested by ELISA. Microtiter plates were coated with GM3(NeuGc) and GM3(NeuAc) (negative control) ganglioside in methanol and specific binding was measured.
**Figure 6:** VH1E10 DNA and deduced amino acid sequences. Sequences are aligned according Kabat's numbering (Kabat and collaborators (1991), Sequences of proteins of immunological interest, Fifth Edition, National Institute of Health), CDRs appeared marked with dotted lines.
**Figure 7:** VK1E10 DNA and deduced amino acid sequences. Sequences are aligned according Kabat's numbering (Kabat et al. (1991), Sequences of proteins of immunological interest, Fifth Edition, National Institute of Health), CDRs appeared marked with dotted lines.
**Figure 8:** VH1E10 was aligned with the most homologous human sequence. Amphipatic segments are underlined and CDRs in bold.
**Figure 9:** VK1E10 was aligned with the most homologous human sequence. Amphipatic segments are underlined and CDRs in bold.
**Figure 10:** Specific binding murine Mab P3 by chimeric Mab 1E10. Different concentrations of Mab 1E10 and MAb C5 (negative control) were tested by ELISA. Microtiter plates were coated with Mab P3 and Mab A3 (negative control) and specific binding was measured.

## Claims

1. A chimeric monoclonal antibody derived from the murine monoclonal antibody Mab P3 that recognizes gangliosides containing N-glycolylated sialic acid, and which is produced by the hybridoma cell line with deposit number ECACC 94113026, wherein the hypervariable domains of the heavy and light chains of Mab P3 comprise the following sequences:
**HEAVY CHAIN**
CDR1: RYSVH
CDR2: MIWGGGSTDYNSALKS
CDR3: SGVREGRAQAWFAY
**LIGHT CHAIN**
CDR1: KASQDVSTAVA
CDR2: SASYRYT
CDR3: QQHYSTPWT;
the framework regions (FRs) of the heavy and light chains of Mab P3 comprise the following sequences:
**HEAVY CHAIN**
FR1: QVQLKESGPGLVAPSQSLSITCTVSGFSLS
FR2: WVRQPPGKGLEWLG
FR3: RLSISKDNSKSQVFLKMNSLQTDDTAMYYCAR
FR4: WGQGTLV
**LIGHT CHAIN**
FR1: DIVMTQSHKFMSTSVGDRVSITC
FR2: WYQQKPGQSPKLLIY
FR3: GVPDRFTGSGSGTDFTFTISSVQAEDLAVYYC
FR4: FGGGTKL;
wherein the chimeric monoclonal antibody, for its humanization and preservation of the binding properties to the antigen, includes the following substitutions in the FR1 region of the light chain:
**LIGHT CHAIN:**
Position 8: His by Pro
Position 9: Lys by Ser
Position 10: Phe by Ser
Position 11: Met by Leu
Position 13: Thr by Ala

2. A monoclonal antibody according to claim 1, wherein the constant region of the heavy chain comprises the amino acid sequence of gamma-1 chain and the constant region of the light chain comprises the amino acid sequence of a kappa chain, both derived from human immunoglobulins.

3. A cell line that produces any of the monoclonal antibodies of claims 1 to 2.

4. A pharmaceutical composition for the treatment of malignant tumors of breast and melanomas, their metastases and relapses which comprises any of the monoclonal antibodies of claims 1 to 2.

5. A pharmaceutical composition for *in vivo* localization and identification of malignant tumors of breast and melanomas, their metastases and relapses which comprises any of the monoclonal antibodies of claims 1 to 2.

6. Use of monoclonal antibody of any one of the claims 1 to 2 for the manufacture of a medicament useful for the treatment of malignant tumours of breast and melanomas, their metastases and relapses.

## Patentansprüche

1. Ein von dem murinen monoklonalen Antikörper Mab P3 abgeteiteter chimärer monoklonaler Antikörper, der Ganglioside erkennt, die N-glykolisierte Sialsäure enthalten, und der hergestellt ist durch die Hybridomzelllinie mit der Hinterlegungsnummer ECACC 94113026, wobei die hypervariablen Domänen der schweren und leichten Ketten von Mab P3 die folgenden Sequenzen enthalten:
SCHWERE KETTE
CDR1:RYSVH
CDR2: MIWGGGSTDYNSALKS
CDR3: SGVREGRAQAWFAY
LEICHTE KETTE
CDR1: KASQDVSTAVA
CDR2:SASYRYT
CDR3: QQHYSTPWT;
die Framework-Bereiche (FRs) der schweren und leichten Ketten von Mab P3 die folgenden Sequenzen enthalten:
SCHWERE KETTE
FR1: QVQLKESGPGLVAPSQSLSITCTVSGFSLS
FR2: WVRQPPGKGLEWLG
FR3: RLSISKDNSKSQVFLKMNSLQTDDTAMYYCAR
FR4: WGQGTLV
LEICHTE KETTE
FR1: DIVMTQSHKFMSTSVGDRVSITC
FR2: WYQQKPGQSPKLLIY
FR3: GVPDRFTGSGSGTDFTFTISSVQAEDLAVYYC
FR4: FGGGTKL;
wobei der chimäre monoklonale Antikörper für seine Humanisierung und Erhaltung der Bindungseigenschaften an das Antigen die folgenden Substitutionen in dem FR1-Bereich der leichten Kette aufweist:
LEICHTE KETTE:
Position 8: His durch Pro
Position 9: Lys durch Ser
Position 10: Phe durch Ser
Position 11: Met durch Leu
Position 13: Thr durch Ala

2. Ein monoklonaler Antikörper nach Anspruch 1, wobei der konstante Bereich der schweren Kette die Aminosäuresequenz einer gamma-1-Kette enthält und der konstante Bereich der leichten Kette die Aminosäuresequenz einer kappa-Kette enthält, die beide von humanen Immunglobulinen abgeleitet sind.

3. Eine Zelllinie, die einen der monoklonalen Antikörper der Ansprüche 1 bis 2 erzeugt.

4. Eine pharmazeutische Zusammensetzung für die Behandlung von bösartigen Tumoren der Brust und Melanomen, ihren Metastasen und Rückfällen, die einen der monoklonalen Antikörper der Ansprüche 1 bis 2 enthält.

5. Eine pharmazeutische Zusammensetzung für *in vivo* Lokalisierung und Identifikation von bösartigen Tumoren der Brust und Melanomen, ihren Metastasen und Rückfällen, die einen der monoklonalen Antikörper der Ansprüche 1 bis 2 enthält.

6. Verwendung von monoklonalem Antikörper nach einem der Ansprüche 1 bis 2 für die Herstellung eines Medikaments, das für die Behandlung von bösartigen Tumoren der Brust und Melanomen, ihren Metastasen und Rückfällen verwendbar ist.

## Revendications

1. Anticorps monoclonal chimère dérivé de l'anticorps monoclonal de souris Mab P3 qui reconnaît les gangliosides contenant de l'acide sialique N-glycosylé, et qui est produit par la lignée cellulaire d'hybridome ayant le numéro de dépôt ECACC 94113026, où les domaines hypervariables des chaînes lourde et légère de Mab P3 comprennent les séquences suivantes:
**Chaîne lourde:**
CDR1: RYSVH
CDR2: MIWGGGSTDYNSALKS
CDR3: SGVREGRAQAWFAY
**Chaîne légère:**
CDR1: KASQDVSTAVA
CDR2: SASYRYT
CDR3: QQHYSTPWT;
les régions charpentes (FR) des chaînes lourde et légère de Mab P3 comprennent les séquences suivantes:
**Chaîne lourde:**
FR1: QVQLKESGPGLVAPSQSLSITCTVSGFSLS
FR2: WVRQPPGKGLEWLG
FR3: RLSISKDNSKSQVFLKMNSLQTDDTAMYYCAR
FR4: WGQGTLV
**Chaîne légère:**
FR1: DIVMTQSHKFMSTSVGDRVSITC
FR2: WYQQKPGQSPKLLIY
FR3: GVPDRFTGSGSGTDFTFTISSVQAEDLAVYYC
FR4: FGGGTKL;
l'anticorps monoclonal chimère comprenant, pour son humanisation et la conservation des propriétés de liaison à l'antigène, les substitutions suivantes dans la région FR1 de la chaîne légère:
**Chaîne légère:**
Position 8: de His par Pro
Position 9: de Lys par Ser
Position 10: de Phe par Ser
Position 11: de Met par Leu
Position 13: de Thr par Ala.

2. Anticorps monoclonal selon la revendication 1, dans lequel la région constante de la chaîne lourde comprend la séquence d'aminoacides d'une chaîne gamma-1 et la région constante de la chaîne légère comprend la séquence d'aminoacides d'une chaîne kappa, provenant toutes les deux d'immunoglobulines humaines.

3. Lignée cellulaire qui produit l'un quelconque des anticorps monoclonaux des revendications 1 à 2.

4. Composition pharmaceutique pour le traitement de tumeurs malignes du sein et de mélanomes, de leurs métastases et de leurs récidives, qui comprend l'un quelconque des anticorps monoclonaux des revendications 1 à 2.

5. Composition pharmaceutique pour la localisation et l'identification *in vivo* de tumeurs malignes du sein et de mélanomes, de leurs métastases et de leurs récidives, qui comprend l'un quelconque des anticorps monoclonaux des revendications 1 à 2.

6. Utilisation d'un anticorps monoclonal selon l'une quelconque des revendications 1 à 2 pour la fabrication d'un médicament destiné au traitement de tumeurs malignes du sein et de mélanomes, de leurs métastases et de leurs récidives.
